# EUROPEAN PATENT SPECIFICATION

(11) **EP 2 129 661 B1**
(45) Date of publication and mention of the grant of the patent: **08.02.2012**
(21) Application number: 07732192.5
(22) Date of filing: 29.03.2007
(51) Int. Cl.: C07D 215/20, C07D 215/22, A61K 31/47, A61P 11/00, A61P 29/00

(54) **QUINOLINE DERIVATIVES AS CRTH2 RECEPTOR LIGANDS**
CHINOLINDERIVATE ALS CRTH2-REZEPTORLIGANDEN
DÉRIVÉS DE QUINOLÉINE COMME LIGANDS DU RÉCEPTEUR CRTH2

(43) Date of publication of application: 09.12.2009
(73) Proprietor: Pulmagen Therapeutics (Asthma) Limited, Fulmer Slough Berkshire SL3 6HD (GB)
(72) Inventor: RAY, Nicholas, Charles, Essex CM19 5TR (GB); FINCH, Harry, Essex CM19 5TR (GB); CRAMP, Michael, Colin, Essex CM19 5TR (GB); ARIENZO, Rosa, Essex CM19 5TR (GB); HYND, George, Essex CM19 5TR (GB); CRACKETT, Peter, Essex CM19 5TR (GB); GRIFFON, Yann, Essex CM19 5TR (GB); HARRISON, Trevor, Keith, Essex CM19 5TR (GB); MONTANA, John, Gary, Essex CM19 5TR (GB)
(74) Representative: Perry, Robert Edward
(86) International application number: PCT/GB2007/001138
(87) International publication number: WO 2008/119917

(56) References cited:
- WO-A-2005/044260
- WO-A-2006/136859
- WO-A-2007/036743

## Description

This invention relates to specific quinoline compounds which are ligands of the CRTH2 receptor (Chemoattractant Receptor-homologous molecule expressed on T Helper cells type 2), and their use in the treatment of diseases responsive to modulation of CRTH2 receptor activity, principally diseases having a significant inflammatory component.

### Background to the Invention

Mast cells are known to play an important role in allergic and immune responses through the release of a number of mediators, such as histamine, leukotrienes, cytokines, prostaglandin D₂, etc (Boyce; Allergy Asthma Proc., 2004, 25, 27-30). Prostaglandin D₂ (PGD₂) is the major metabolite produced by the action of cyclooxygenase on arachadonic acid by mast cells in response to allergen challenge (Lewis et al; J. Immunol., 1982, 129, 1627-1631). It has been shown that PGD₂ production is increased in patients with systemic mastocytosis (Roberts; N. Engl. J. Med., 1980, 303, 1400-1404), allergic rhinitis (Naclerio et al; Am. Rev. Respir. Dis., 1983, 128, 597-602; Brown et al; Arch. Otolarynol. Head Neck Surg., 1987, 113, 179-183; Lebel et al; J. Allergy Clin. Immunol., 1988, 82, 869-877), bronchial asthma (Murray et al; N. Engl. J. Med., 1986, 315, 800-804; Liu et al; Am. Rev. Respir. Dis., 1990, 142, 126-132; Wenzel et al; J. Allergy Clin. Immunol., 1991, 87, 540-548), and urticaria (Heavey et al; J. Allergy Clin. Immunol., 1986, 78, 458-461). PGD₂ mediates it effects through two receptors, the PGD₂ (or DP) receptor (Boie et al; J. Biol. Chem., 1995, 270, 18910-18916) and the chemoattractant receptor-homologous molecule, expressed on Th2 (or CRTH2) (Nagata et al; J. Immunol., 1999, 162, 1278-1289; Powell; Prostaglandins Luekot. Essent. Fatty Acids, 2003, 69, 179-185). Therefore, it has been postulated that agents that antagonise the effects of PGD₂ at its receptors may have beneficial effects in number of disease states.

The CRTH2 receptor has been shown to be expressed on cell types associated with allergic inflammation, such as basophils, eosinophils, and Th2-type immune helper cells (Hirai et al; J. Exp. Med., 2001, 193, 255-261). The CRTH2 receptor has been shown to mediate PGD₂-mediated cell migration in these cell types (Hirai et al; J. Exp. Med., 2001, 193, 255-261), and also to play a major role in neutrophil and eosinophil cell recruitment in a model of contact dermatitis (Takeshita et al; Int. Immunol., 2004, 16, 947-959). Ramatroban {(3R)-3-[(4-fluorophenyl)sulphonyl-amino]-1,2,3,4-tetrahydro-9H-carbazole-9-propanoic acid}, a dual CRTH2 and thromboxane A₂ receptor antagonist, has been shown to attenuate these responses (Sugimoto et al; J. Pharmacol. Exp. Ther, 2003, 305, 347-352; Takeshita et al; *op. cit*.). The potential of PGD₂ both to enhance allergic inflammation and induce an inflammatory response has been demonstrated in mice and rats. Transgenic mice over expressing PGD₂ synthase exhibit an enhanced pulmonary eosinophilia and increased levels of Th2 cytokines in response to allergen challenge (Fujitani et al; J. Immunol., 2002, 168, 443-449). In addition, exogenously administered CRTH2 agonists enhance the allergic response in sensitised mice (Spik et al; J. Immunol., 2005, 174, 3703-3708). In rats exogenously applied CRTH2 agonists cause a pulmonary eosinophilia but a DP agonist (BW 245C) or a TP agonist (I-BOP) showed no effect (Shirashi et al; J. Pharmacol. Exp Ther., 2005, 312, 954-960). These observations suggest that CRTH2 antagonists may have valuable properties for the treatment of diseases mediated by PGD₂.

Our copending International Patent Application No. PCT/GB2006/003644 describes the use, for the manufacture of a medicament for use in the treatment of conditions responsive to modulation of CRTH2 receptor activity, of a compound of formula [1] or a pharmaceutically acceptable salt, N-oxide, hydrate or solvate thereof: 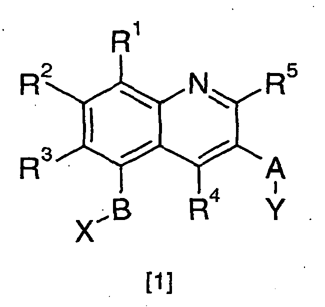 in which:
R¹, R², R³, R⁴ and R⁵ are independently hydrogen, C₁-C₆alkyl, fully or partially fluorinated C₁-C₆alkyl, cyclopropyl, halo, -S(O)ₙR⁶, -SO₂NR⁷R⁸, -NR⁷R⁸, -NR⁷C(O)R⁶, -CO₂R⁷, -C(O)NR⁷R⁸, -C(O)R⁶, -NO₂, -CN or a group -OR⁹;
   wherein each R⁶ is independently C₁-C₆alkyl, fully or partially fluorinated C₁-C₆alkyl, cycloalkyl, aryl, or heteroaryl;
   R⁷, R⁸ are independently C₁-C₆alkyl, fully or partially fluorinated C₁-C₆alkyl, cycloalkyl, cycloalkyl-(C₁-C₆alkyl)-, aryl, heteroaryl or hydrogen;
   R⁹ is hydrogen, C₁-C₆alkyl, fully or partially fluorinated C₁-C₆alkyl, cycloalkyl, cycloalkyl-(C₁-C₆alkyl)-, or a group -SO₂R⁶;
A is -CHR¹⁰-, -C(O)-, -S(O)ₙ₋, -O-, or -NR¹⁰- wherein n is an integer from 0-2 and R¹⁰ is hydrogen, C₁-C₃alkyl, or fully or partially fluorinated C₁-C₃alkyl group;
B is a direct bond, or a divalent radical selected from -CH₂-, -CH₂CH₂-, -CHR¹¹-, -CR¹¹R¹²-, -CH₂CHR¹¹- in either orientation, -CH₂CR¹¹R¹²- in either orientation, -CHR¹¹CHR¹²- in either orientation, and divalent radicals of formula -(CR¹¹R¹²)ₚ-Z-wherein Z is attached to the ring carrying R¹, R² and R³; wherein
   R¹¹ is C₁-C₃alkyl, cyclopropyl, or fully or partially fluorinated C₁-C₃alkyl;
   R¹² is methyl or fully or partially fluorinated methyl;
   p is independently 1 or 2; and
   Z is -O-, -NH-, or -S(O)ₙ-, wherein n is an integer from 0-2;
X is a carboxylic acid, tetrazole, 3-hydroxyisoxazole, hydroxamic acid, phosphinate, phosphonate, phosphonamide, or sulfonic acid group, or a group of formula C(=O)NHSO₂R⁶ or SO₂NHC(=O)R⁶;
Y is aryl, heteroaryl, aryl-fused-heterocycloalkyl, heteroaryl-fused-cycloalkyl, heteroaryl-fused-heterocycloalkyl or aryl-fused-cycloalkyl group.

### Detailed Description of the Invention

The present invention provides a group of specific compounds of the same structural class as those with which our copending application PCT/GB2006/003644 referred to above is concerned, but not specifically disclosed therein.

The invention provides a compound selected from the group consisting of
[3-(4-cyanobenzyl)-4-difluoromethoxy-2-ethyl-8-fluoroquinolin-5-yloxy]acetic acid,
[3-(4-bromobenzyl)-4-difluoromethoxy-2-ethyl-8-fluoroquinolin-5-yloxy]acetic acid,
[4-ethyl-8-fluoro-3-(4-methanesulfonylbenzyl)-2-methylquinolin-5-yloxy]acetic acid,
[2-ethyl-8-fluoro-3-(4-methanesulfonylbenzyl)-4-methylquinolin-5-yloxy]acetic acid,
[4-difluoromethoxy-2-ethyl-8-fluoro-3-(4-methoxycarbonylaminobenzyl)quinolin-5-yloxy]acetic acid,
[4-difluoromethoxy-2-ethyl-8-fluoro-3-(3-methanesulfonylbenzyl)quinolin-5-yloxy]acetic acid,
(S)-2-[3-(4-chlorobenzyl)-4-difluoromethoxy-2-ethyl-8-fluoroquinolin-5-yloxy]propionic acid,
{4-difluoromethoxy-2-ethyl-8-fluoro-3-[4-(1-hydroxy-1-methylethyl)benzyl]quinolin-5-yloxy)acetic acid,
[8-chloro-3-(4-cyanobenzyl)-4-difluoromethoxy-2-ethylquinolin-5-yloxy]acetic acid,
[8-chloro-3-(2-chloro-4-cyanobenzyl)-4-difluoromethoxy-2-ethylquinolin-5-yloxy]acetic acid,
[8-chloro-3-(4-cyanobenzyl)-2-difluoromethoxy-4-methylquinolin-5-yloxy]acetic acid,
[3-(4-cyanobenzyl)-2-difluoromethoxy-8-fluoro-4-methylquinolin-5-yloxy]acetic acid,
and salts, N-oxides, hydrates and solvates thereof.

The compounds with which the invention is concerned are CRTH2 receptor antagonists.

A second aspect of the invention is the aforementioned compounds for use in a method of treatment of conditions responsive to modulation of CRTH2 receptor activity, comprising administering to a patient suffering such disease an effective amount of a compound of the invention, or a pharmaceutically acceptable salt, N-oxide, hydrate or solvate thereof.

Examples of such diseases include asthma, rhinitis, allergic airway syndrome, allergic rhinobronchitis, bronchitis, chronic obstructive pulmonary disease (COPD), nasal polyposis, sarcoidosis, farmer's lung, fibroid lung, cystic fibrosis, chronic cough, conjunctivitis, atopic dermatitis, Alzheimer's disease, amyotrophic lateral sclerosis, AIDS dementia complex, Huntington's disease, frontotemporal dementia, Lewy body dementia, vascular dementia, Guillain-Barre syndrome, chronic demyelinating polyradiculoneurophathy, multifocal motor neuropathy, plexopathy, multiple sclerosis, encephalomyelitis, panencephalitis, cerebellar degeneration and encephalomyelitis, CNS trauma, migraine, stroke, rheumatoid arthritis, ankylosing spondylitis, Behçet's Disease, bursitis, carpal tunnel syndrome, inflammatory bowel disease, Crohn's disease, ulcerative colitis, dermatomyositis, Ehlers-Danlos Syndrome (EDS), fibromyalgia, myofascial pain, osteoarthritis (OA), osteonecrosis, psoriatic arthritis, Reiter's syndrome (reactive arthritis), sarcoidosis, scleroderma, Sjogren's Syndrome, soft tissue disease, Still's Disease, tendinitis, polyarteritis Nodossa, Wegener's Granulomatosis, myositis (polymyositis dermatomyositis), gout, atherosclerosis, lupus erythematosus, systemic lupus erythematosus (SLE), type I diabetes, nephritic syndrome, glomerulonephritis, acute and chronic renal failure, eosinophilia fascitis, hyper IgE syndrome, sepsis, septic shock, ischemic reperfusion injury in the heart, allograft rejection after transplantations, and graft versus host disease.

However, the compounds with which the invention is concerned are primarily of value for the treatment of asthma, chronic obstructive pulmonary disease, rhinitis, allergic airway syndrome, or allergic rhinobronchitis. Psoriasis, atopic and non-atopic dermatitis Crohn's disease, ulcerative colitis, and irritable bowel disease are other specific conditions where the present compounds may have particular utility.

As used herein the term "salt" includes base addition, acid addition and quaternary salts. Compounds of the invention which are acidic can form salts, including pharmaceutically acceptable salts, with bases such as alkali metal hydroxides, e.g. sodium and potassium hydroxides; alkaline earth metal hydroxides e.g. calcium, barium and magnesium hydroxides; with organic bases e.g. N-methyl-D-glucamine, choline tris(hydroxymethyl)amino-methane, L-arginine, L-lysine, N-ethyl piperidine, dibenzylamine and the like. Specific salts with bases include the benzathine, calcium, diolamine, meglumine, olamine, potassium, procaine, sodium, tromethamine and zinc salts. Those compounds of the invention which are basic can form salts, including pharmaceutically acceptable salts with inorganic acids, e.g. with hydrohalic acids such as hydrochloric or hydrobromic acids, sulphuric acid, nitric acid for phosphoric acid and the like, and with organic acids e.g. with acetic, tartaric, succinic, fumaric, maleic, malic, salicylic, citric, methanesulphonic, p-toluenesulphonic, benzoic, benzenesunfonic, glutamic, lactic, and mandelic acids and the like. Where a compound contains a quaternary ammonium group acceptable counter-ions may be, for example, chlorides, bromides, sulfates, methanesulfonates, benzenesulfonates, toluenesulfonates (tosylates), napadisylates (naphthalene-1,5-disulfonates or naphthalene-1-(sulfonic acid)-5-sulfonates), edisylates (ethane-1,2-disulfonates or ethane-1-(sulfonic acid)-2-sulfonates), isethionates (2-hydroxyethylsulfonates), phosphates, acetates, citrates, lactates, tartrates, mesylates, maleates, malates, fumarates, succinates, xinafoates, p-acetamidobenzoates and the like; wherein the number of quaternary ammonium species balances the pharmaceutically acceptable salt such that the compound has no net charge.

### Compositions

As mentioned above, the compounds with which the invention is concerned are CRTH2 receptor antagonists, and are useful in the treatment of diseases which benefit from such modulation. Examples of such diseases are referred to above, and include asthma, rhinitis, allergic airway syndrome, and allergic rhinobronchitis.

It will be understood that the specific dose level for any particular patient will depend upon a variety of factors including the activity of the specific compound employed, the age, body weight, general health, sex, diet, time of administration, route of administration, rate of excretion, drug combination and the severity of the particular disease undergoing treatment. Optimum dose levels and frequency of dosing will be determined by clinical trial, as is required in the pharmaceutical art. In general, the daily dose range will lie within the range of from about 0.001 mg to about 100 mg per kg body weight of a mammal, often 0.01 mg to about 50 mg per kg, for example 0.1 to 10 mg per kg, in single or divided doses. On the other hand, it may be necessary to use dosages outside these limits in some cases.

The compounds with which the invention is concerned may be prepared for administration by any route consistent with their pharmacokinetic properties. Orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; nonaqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin, the drug may be made up into a cream, lotion or ointment. Cream or ointment formulations which may be used for the drug are conventional formulations well known in the art, for example as described in standard textbooks of pharmaceutics such as the British Pharmacopoeia.

The drug may also be formulated for inhalation, for example as a nasal spray, or dry powder or aerosol inhalers. For delivery by inhalation, the active compound is preferably in the form of microparticles. They may be prepared by a variety of techniques, including spray-drying, freeze-drying and micronisation. Aerosol generation can be carried out using, for example, pressure-driven jet atomizers or ultrasonic atomizers, preferably using propellant-driven metered aerosols or propellant-free administration of micronized active compounds from, for example, inhalation capsules or other "dry power delivery systems.

The active ingredient may also be administered parenterally in a sterile medium. Depending on the vehicle and concentration used, the drug can either be suspended or dissolved in the vehicle. Advantageously, adjuvants such as a local anaesthetic, preservative and buffering agents can be dissolved in the vehicle.

Other compounds may be combined with compounds of this invention for the prevention and treatment of prostaglandin-mediated diseases. Thus the present invention is also concerned with pharmaceutical compositions for preventing and treating PGD₂-mediated diseases comprising a therapeutically effective amount of a compound of the invention and one or more other therapeutic agents. Suitable therapeutic agents for a combination therapy with compounds of the invention include, but are not limited to: (1) corticosteroids, such as fluticasone, budesonide or ciclesonide; (2) β2-adrenoreceptor agonists, such as salmeterol, formeterol or indacaterol; (3) leukotriene modulators, for example leukotriene antagonists such as montelukast or pranlukast or leukotriene biosynthesis inhibitors such as Zileuton or BAY-x1005; (4) anticholinergic agents, for example muscarinic-3 (M₃) receptor antagonists such as tiotropium bromide; (5) phosphodiesterase-IV (PDE-IV) inhibitors, such as roflumilast or cilomilast; (6) antihistamines, for example selective histamine-1 (H1) receptor antagonists, such as loratidine or astemizole; (7) antitussive agents, such as codeine or dextramorphan; (8) non-selective COX-1 /COX-2 inhibitors, such as ibuprofen or ketoprofen; (9) COX-2 inhibitors, such as celecoxib and rofecoxib; (10) VLA-4 antagonists, such as those described in WO97/03094 and WO97/02289; (11) TNF-α inhibitors, for example anti-TNF monoclonal antibodies, such as Remicade and CDP-870 and TNF receptor immunoglobulin molecules, such as Enbrel; (12) inhibitors of matrix metalloprotease (MMP), for example MMP8, 9 and 12; (13) human neutrophil elastase inhibitors, such as those described in WO2005/026124 and WO2003/053930; (14) Adenosine A2a agonists such as those described in EP1052264 and EP1241176 (15) Adenosine A2b antagonists such as those described in WO2002/42298; (16) modulators of chemokine receptor function, for example antagonists of CCR3 and CCR8; (17) compounds which modulate the action of other prostanoid receptors, for example a PGD2 (DP) receptor antagonist or a thromboxane A2 antagonist; and (18) compounds which modulate Th2 function, for example, PPAR agonists.

The weight ratio of the compound of the invention to the second active ingredient may be varied and will depend upon the effective dose of each ingredient. Generally, an effective dose of each will be used.

The following examples describe the preparation of compounds of the invention:

### Examples

The invention will now be described with reference to the following examples. It will be appreciated that the invention is described by way of example only'and modification of detail may be made without departing from the scope of the invention, which is defined by the claims.

¹H NMR spectra were recorded at ambient temperature using a Varian Unity Inova (400MHz) spectrometer with a triple resonance 5 mm probe spectrometer. Chemical shifts are expressed in ppm relative to tetramethylsilane. The following abbreviations have been used: br s = broad singlet, s = singlet, d = doublet, dd = double doublet, t = triplet, q = quartet, m = multiplet.

Mass Spectrometry (LCMS) experiments to determine retention times and associated mass ions were performed using the following methods:

Method A: experiments were performed on a Micromass Platform LCT spectrometer with positive ion electrospray and single wavelength UV 254 nm detection using a Higgins Clipeus C18 5 µm 100 x 3.0 mm column and a 2 mL / minute flow rate. The initial solvent system was 95 % water containing 0.1 % formic acid (solvent A) and 5 % acetonitrile containing 0.1.% formic acid (solvent B) for the first minute followed by a gradient up to 5 % solvent A and 95 % solvent B over the next 14 minutes. The final solvent system was held constant for a further 2 minutes.

Method B: experiments were performed on a Micromass Platform LC spectrometer with positive and negative ion electrospray and ELS / Diode array detection using a Phenomenex Luna C18(2) 30 x 4.6 mm column and a 2 mL / minute flow rate. The solvent system was 95 % solvent A and 5 % solvent B for the first 0.50 minutes followed by a gradient up to 5 % solvent A and 95 % solvent B over the next 4 minutes. The final solvent system was held constant for a further 0.50 minutes

Reverse-phase preparative HPLC purifications were carried out using Genesis 7 micron C-18 bonded silica stationary phase in columns 10 cm in length and 2 cm internal diameter. The mobile phase used was mixtures of acetonitrile and water (both buffered with 0.1 % v/v formic acid) with a flow rate of 10 mL per minute and typical gradients of 40 to 90 % organic modifier ramped up over 30 to 40 minutes. Fractions containing the required product (identified by LC-MS analysis) were pooled, the organic fraction removed by evaporation, and the remaining aqueous fraction lyophilised, to give the final product.

### Example 1: [3-(4-cyanobenzyl)-4-difluoromethoxy-2-ethyl-8-fluoroquinolin-5-yloxy]acetic acid

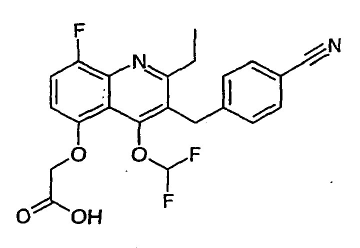

### Preparation 1a: (3-amino-4-fluorophenoxy)acetic acid methyl ester

3-Amino-4-fluorophenol (3.0 g) was added to a stirred suspension of sodium hydride (60 % in oil, 0.94 g) in *N,N*-dimethylformamide (30 mL) at 0 °C. The mixture was warmed to room temperature for 15 minutes and then cooled to 0 °C and this mixture was treated with bromoacetic acid methyl ester (3.3 g). The resulting mixture was warmed to room temperature and then stirred at this temperature for 2 hours. The mixture was treated with dilute aqueous ammonium chloride solution and extracted with ethyl acetate. The combined extracts were washed with saturated aqueous sodium chloride solution, dried over magnesium sulfate and the solvent removed under reduced pressure. Purification of the residue by column chromatography on silica gel, eluting with a mixture of toluene, dichloromethane and ethyl acetate (2:1:0 to 0:1:0 to 0:20:1 by volume) gave title compound, 2.7 g.
¹H NMR (DMSO-d6): δ 3.70 (s, 3H), 4.65 (s, 2H), 5.15 (br s, 2H), 6.00 (dt, J = 3.1, 8.8 Hz, 1H), 6.30 (dd, J = 3.1, 7.6 Hz, 1H), 6.85 (dd, J = 8.8, 11.2 Hz, 1H). MS: ESI (+ve) (Method B): 200 (M+H)⁺, Retention time 2.5 min.

### Preparation 1b: 2-(4-cyanobenzyl)-3-oxopentanoic acid ethyl ester

A suspension of potassium *tert*-butoxide (4.7 g) in tetrahydrofuran (150 mL) at 0 °C was treated with a mixture of *tert*-butanol (5.0 mL) and 3-oxobutyric acid ethyl ester (5.0 mL) and the resulting mixture was stirred at room temperature for 45 minutes. The mixture was treated with a solution of 4-bromomethylbenzonitrile (6.9 g) in tetrahydrofuran (50 mL) and the resulting mixture was heated at 70°C for 20 hours. The mixture was cooled to room temperature, diluted with water and the tetrahydrofuran removed under reduced pressure. The residue was diluted with water, extracted with ethyl acetate and the combined extracts washed with saturated aqueous sodium chloride solution and then dried over magnesium sulfate. The solvent was removed under reduced pressure and the residue triturated with diethyl ether to afford title compound as an off-white solid, 2.9 g.
MS: ESI (+ve) (Method B): 260 (M+H)⁺, Retention time 3.2 min.

### Preparation 1c: [3-(4-cyanobenzyl)-2-ethyl-8-fluoro-4-hydroxyquinolin-5-yloxy]acetic acid methyl ester

A mixture of (3-amino-4-fluorophenoxy)acetic acid methyl ester (0.90 g), 2-(4-cyanobenzyl)-3-oxopentanoic acid ethyl ester (1.2 g), polyphosphoric acid (5.0 g) and 1,4-dioxane (30 mL) was heated at 100°C for 18 hours. The mixture was cooled to room temperature, diluted with water and extracted with ethyl acetate. The combined extracts were washed with water, dried over magnesium sulfate and concentrated under reduced pressure. Purification of the residue by column chromatography on silica gel, eluting with a mixture of cyclohexane and ethyl acetate gave title compound, 0.18 g.
MS: ESI (+ve) (Method B): 395 (M+H)⁺, Retention time 2.9 min.

### Preparation 1 d: [3-(4-cyanobenzyl)-4-difluoromethoxy-2-ethyl-8-fluoroquinolin-5-yloxy]acetic acid methyl ester

A mixture of [3-(4-cyanobenzyl)-2-ethyl-8-fluoro-4-hydroxyquinolin-5-yloxy)acetic acid methyl ester (0.17 g), *N,N*-dimethylformamide (5.0 mL), potassium carbonate (0.090 g) and acetic acid chlorodifluoromethyl ester (0.14 mL) was stirred at 80 °C for 6 hours. The mixture was cooled to room temperature, diluted with water and extracted with ethyl acetate and the combined extracts dried over magnesium sulfate. The solvent was removed under reduced pressure and the residue purified by column chromatography on silica gel, eluting with a mixture of cyclohexane and ethyl acetate. Further purification by column chromatography on silica gel, eluting with a mixture of dichloromethane and ethyl acetate gave title compound, 0.12 g.
¹H NMR (CDCl₃): δ 1.30 (t, J = 7.5 Hz, 3H), 2.85 (q, J = 7.5 Hz, 2H), 3.85 (s, 3H), 4.45 (s, 2H), 4.80 (s, 2H), 6.70 (dd, J = 3.7, 8.7 Hz, 1H), 7.00 (t, J = 75 Hz, 1H), 7.20 (d, J = 8.5 Hz, 2H), 7.30 (dd, J = 8.7, 9.7 Hz, 1H), 7.55 (d, J = 8.5 Hz, 2H).
MS: ESI (+ve) (Method B): 445 (M+H)⁺, Retention time 3.6 min.

### Preparation 1e: [3-(4-cyanobenzyl)-4-difluoromethoxy-2-ethyl-8-fluoroquinolin-5-yloxy]acetic acid

A solution of [3-(4-cyanobenzyl)-4-difluoromethoxy-2-ethyl-8-fluoroquinolin-5-yloxy]acetic acid methyl ester (0.12 g) in tetrahydrofuran (5.0 mL) was treated with 1.0 M aqueous lithium hydroxide solution (0.52 mL) and the resulting mixture was stirred at room temperature for 1 hour. The organic solvent was removed under reduced pressure and the pH of the residue adjusted to 4-5 by the addition of 1.0 M aqueous hydrochloric acid. The mixture was extracted with ethyl acetate and the combined extracts dried over magnesium sulfate. The solvent was removed under reduced pressure and the residue purified by column chromatography on C-18 column, eluting with a mixture of water and methanol to afford title compound, 0.065 g.
¹H NMR (CDCl₃): δ 1.25 (t, J = 7.4 Hz, 3H), 2.90 (q, J = 7.4 Hz, 2H), 4.45 (s, 2H), 4.85 (s, 2H), 6.75 (dd, J = 3.6, 8.8 Hz, 1H), 6.90 (t, J = 75 Hz, 1H), 7.20 (d, J = 8.5 Hz, 2H), 7.35 (dd, J = 8.8, 9.6 Hz, 1H), 7.55 (d, J = 8.5 Hz, 2H).
MS: ESI (+ve) (Method A): 431 (M+H)⁺, Retention time 10.9 min.
MS: ESI (+ve) (Method B): 431 (M+H)⁺, Retention time 3.3 min.

### Example 2: [3-(4-bromobenzyl)-4-difluoromethoxy-2-ethyl-8-fluoroquinolin-5-yloxy]acetic acid

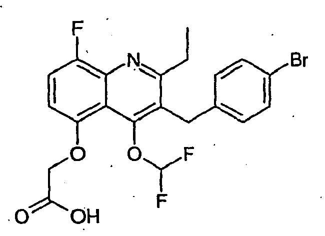

### Preparation 2a: 2-(4-bromobenzyl)-3-oxopentanoic acid ethyl ester

The title compounds were prepared by the method of Preparation 1b using (3-oxobutyric acid ethyl ester and 1-bromo-4-bromomethylbenzene.
¹H NMR (CDCl₃): δ 1.05 (t, J = 7.3 Hz, 3H), 1.25 (t, J = 7.1 Hz, 3H), 2.35 (m, 1H), 2.60 (m, 1H), 3.10 (m, 2H), 3.75 (t, J = 7.6 Hz, 1H), 4.15 (m, 2H), 7.05 (d, J = 8.5 Hz, 2H), 7.40 (d, J = 8.5 Hz, 2H).

### Preparation 2b: [3-(4-bromobenzyl)-2-ethyl-8-fluoro-4-hydroxyquinolin-5-yloxy]acetic acid methyl ester

The title compound was prepared by the method of Preparation 1 c using (3-amino-4-fluorophenoxy)acetic acid methyl ester and 2-(4-bromobenzyl)-3-oxopentanoic acid ethyl ester.
MS: ESI (+ve) (Method B): 448 (M+H)⁺, Retention time 3.2 min.

### Preparation 2c: [3-(4-bromobenzyl)-4-difluoromethoxy-2-ethyl-8-fluoroquinolin-5-yloxy]acetic acid methyl ester

The title compounds were prepared by the method of Preparation 1 d using [3-(4-bromobenzyl)-2-ethyl-8-fluoro-4-hydroxyquinolin-5-yloxy]acetic acid methyl ester and acetic acid chlorodifluoromethyl ester.
MS: ESI (+ve) (Method B): 498 (M+H)⁺, Retention time 4.1 min.

### Preparation 2d: [3-(4-bromobenzyl)-4-difluoromethoxy-2-ethyl-8-fluoroquinolin-5-yloxy]acetic acid

A mixture of [3-(4-bromobenzyl)-4-difluoromethoxy-2-ethyl-8-fluoroquinolin-5-yloxy]acetic acid methyl ester (0.20 g), methanol (8.0 mL) and water (0.32 mL) was treated with 5.0 M aqueous lithium hydroxide solution (0.40 mL) and the resulting mixture was stirred at room temperature for 30 minutes. The mixture was acidified by the addition of glacial acetic acid and concentrated under reduced pressure. The residue was diluted with water and the resulting precipitate collected by filtration, washed with water and then dried to afford title compound as a pale primrose solid, 0.13 g.
¹H NMR (DMSO-d6): δ 1.20 (t, J = 7_{.}4 Hz, 3H), 2.75 (q, J = 7.4 Hz, 2H), 4.25 (s, 2H), 4.50 (s, 2H), 6.80 (dd, J = 3.7, 5.1 Hz, 1H), 7.00 (d, J = 8.5 Hz, 2H), 7.40 (m, 3H), 7.80 (t, J = 75 Hz, 1H).
MS: ESI (+ve) (Method A): 484 (M+H)⁺, Retention time 12.7 min.
MS: ESI (+ve) (Method B): 484 (M+H)⁺, Retention time 3.8 min.

### Example 3 and 4: [4-ethyl-8-fluoro-3-(4-methanesulfonylbenzyl)-2-methylquinolin-5-yloxy]acetic acid and [2-ethyl-8-fluoro-3-(4-methanesulfonylbenzyl)-4-methylquinolin-5-yloxy]acetic acid

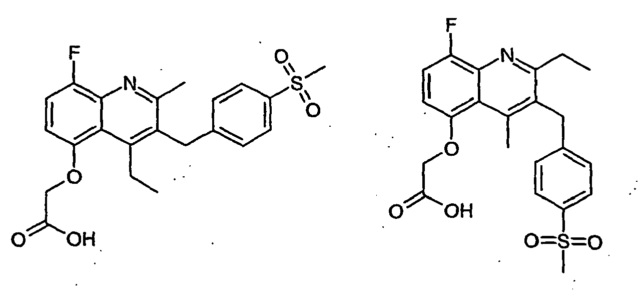

### Preparation 3a and 4a: 3-(4-methanesulfonylbenzyl)hexane-2,4-dione

A solution of hexane-2,4-dione (3.5 g) in *N,N*-dimethylformamide (5.0 mL) was added dropwise over a period of 15 minutes to a stirred suspension of sodium hydride (60 % in oil, 1.1 g) in *N,N*-dimethylflormamide (30 mL) at 0 °C. The mixture was warmed to room temperature, cooled to -10°C and then treated with a solution of 1-bromomethyl-4-methanesulfonylbenzene (8.9 g) in *N,N*-dimethylformamide (15 mL). The resulting mixture was warmed to room temperature and then stirred at this temperature overnight. The mixture was diluted with 1.0 M aqueous hydrochloric acid (100 mL) and water (200 mL) and then extracted with ethyl acetate. The combined extracts were washed with a saturated aqueous sodium chloride solution, dried over magnesium sulfate and concentrated under reduced pressure. Purification of the residue by column chromatography on silica gel, eluting with a mixture of toluene and ethyl acetate (1:0 to 0:1 by volume) gave title compound as a colourless oil, 3.6 g.
MS: ESI (-ve) (Method B): 281 (M-H)⁻, Retention time 2.7 min.

### Preparation 3b and 4b: 4-ethyl-8-fluoro-3-(4-methanesulfonylbenzyl)-2-methylquinolin-5-ol and 2-ethyl-8-fluoro-3-(4-methanesulfonylbenzyl)-4-methylquinolin-5-ol

A mixture of 3-amino-4-fluorophenol (0.81 g), 3-(4-methanesulfonylbenzyl)hexane-2,4-dione (1.8 g) and *p*-toluenesulfonic acid monohydrate (0.1 g) was heated at 150 °C for 1.5 hours and then left to strand at room temperature overnight. The mixture was treated with a further portion of 3-amino-4-fluorophenol (0.81 g), heated at 150 °C for 2 hours and then cooled to room temperature. Purification by column chromatography on silica gel, eluting with a mixture of toluene and ethyl acetate gave 4-ethyl-8-fluoro-3-(4-methanesulfonylbenzyl)-2-methylquinolin-5-ol as a honey coloured gum/glass, 0.22 g and 2-ethyl-8-fluoro-3-(4-methanesulfonylbenzyl)-4-methylquinolin-5-ol as a honey coloured gum/glass, 0.73 g.
4-Ethyl-8-fluoro-3-(4-methanesulfonylbenzyl)-2-methylquinolin-5-ol
MS: ESI (+ve) (Method B): 374 (M+H)⁺, Retention time 2.3 min.
2-Ethyl-8-fluoro-3-(4-methanesulfonylbenzyl)-4-methylquinolin-5-ol
MS: ESI (+ve) (Method B): 374 (M+H)⁺, Retention time 2.4 min.

### Preparation 3c: [4-ethyl-8-fluoro-3-(4-methanesulfonylbenzyl)-2-methyl-quinolin-5-yloxy]acetic acid methyl ester

A solution of 4-ethyl-8-fluoro-3-(4-methanesulfonylbenzyl)-2-methylquinolin-5-ol (0.22 g) in *N,N*-dimethylformamide (4.0 mL) was treated with potassium carbonate (0.12 g) and bromoacetic acid methyl ester (0.078 mL) and the resulting mixture was stirred at room temperature for 2 hours. The mixture was diluted with water, extracted with ethyl acetate and the combined extracts were washed with saturated aqueous sodium chloride solution and then dried over magnesium sulfate. The solvent was removed under reduced pressure and the residue purified by column chromatography on silica gel, eluting with a mixture of cyclohexane and ethyl acetate (1:0 to 0:1 by volume) to afford title compound as a honey coloured gum, 0.083 g.
¹H NMR (CDCl₃): δ 1.25 (t, J = 7.2 Hz, 3H), 2.65 (s, 3H), 3.05 (s, 3H), 3.35 (q, J = 7.2 Hz, 2H), 3.85 (s, 3H), 4.40 (s, 2H), 4.75 (s, 2H), 6.65 (dd, J = 4.0, 8.7 Hz, 1H), 7.20-7.25 (m, 3H), 7.85 (d, J = 8.4 Hz, 2H).

### Preparation 4c: [2-ethyl-8-fluoro-3-(4-methanesulfonylbenzyl)-4-methylquinolin-5-yloxy]acetic acid methyl ester

The title compound was prepared by the method of Preparation 3c using 2-ethyl-8-fluoro-3-(4-methanesulfonylbenzyl)-4-methylquinolin-5-ol and bromoacetic acid methyl ester.
MS: ESI (+ve) (Method B): 446 (M+H)⁺, Retention time 3.6 min.

### Preparation 3d: [4-ethyl-8-fluoro-3-(4-methanesulfonylbenzyl)-2-methylquinolin-5-yloxy]acetic acid

A solution of [4-ethyl-8-fluoro-3-(4-methanesulfonylbenzyl)-2-methyl-quinolin-5-yloxy]acetic acid methyl ester (0.080 g) in methanol (5.0 mL) and water (0.50 mL) was treated with 5.0 M aqueous lithium hydroxide solution (0.25 mL) and the resulting solution was stirred at room temperature for 2 hours. The mixture was acidified by the addition of glacial acetic acid, concentrated under reduced pressure and then partitioned between ethyl acetate and water. The organic phase was dried over magnesium sulfate and concentrated under reduced pressure. The residue was purified by preparative reverse-phase HPLC to afford title compound as a primrose powder, 0.031 g.
¹H NMR (DMSO-d6): δ 1.10 (t, J = 7.3 Hz, 3H), 2.50 (s, 3H), 3.15 (s, 3H), 3.30 (q, 2H), 4.35 (s, 2H), 4.80 (s, 2H), 6.80 (dd, J = 4.0, 8.7 Hz, 1H), 7.25 (d, J = 8.3 Hz, 2H), 7.35 (dd, J = 8.7,10.0 Hz, 1H), 7.80 (d, J = 8.3 Hz, 2H).
MS: ESI (+ve) (Method A): 432 (M+H)⁺, Retention time 7.3 min.
MS: ESI (+ve) (Method B): 432 (M+H)⁺, Retention time 2.6 min.

### Preparation 4d: [2-ethyl-8-fluoro-3-(4-methanesulfonylbenzyl)-4-methylquinolin-5-yloxy]acetic acid

A solution of 2-ethyl-8-fluoro-3-(4-methanesulfonylbenzyl)-4-methylquinolin-5-ol (0.41 g) in methanol (15 mL) and water (1.5 mL) was treated with 5.0 M aqueous lithium hydroxide solution (0.75 mL) and the resulting solution was stirred at room temperature for 2 hours. The mixture was acidified by the addition of glacial acetic acid, concentrated under reduced pressure and partitioned between ethyl acetate and water. The organic phase was dried over magnesium sulfate and concentrated under reduced pressure. The residue was purified by preparative reverse-phase HPLC to afford title compound as a white powder, 0.082 g.
¹H NMR (DMSO-d6): δ 1.15 (t, J = 7.4 Hz, 3H), 2.75 (s, 3H), 2.85 (q, J = 7.4 Hz, 2H), 3.15 (s, 3H), 4.40 (s, 2H), 4.75 (s, 2H), 6.85 (dd, J = 4.0, 8.7 Hz, 1H), 7.25 (d, J = 8.4 Hz, 2H), 7.35 (dd, J = 8.7, 10.1 Hz, 1H), 7.80 (d, J = 8.4 Hz, 2H), 13.0 (br s, 1H).
MS: ESI (+ve) (Method A): 432 (M+H)⁺, Retention time 10.9 min.
MS: ESI (+ve) (Method B): 432 (M+H)⁺, Retention time 3.3 min.

### Example 5: [4-difluoromethoxy-2-ethyl-8-fluoro-3-(4 methoxycarbonylaminobenzyl)quinolin-5-yloxy]acetic acid

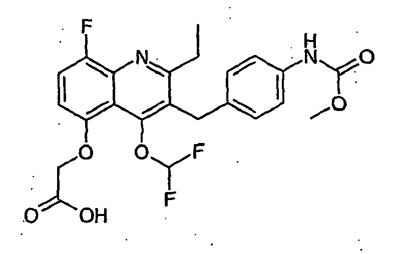

### Preparation 5a: [4-difluoromethoxy-2-ethyl-8-fluoro-3-(4-methoxycarbonylaminobenzyl)quinolin-5-yloxy]acetic acid methyl ester

A mixture of [3-(4-bromobenzyl)-4-difluoromethoxy-2-ethyl-8-fluoroquinolin-5-yloxy]acetic acid methyl ester (0.30 g), carbamic acid methyl ester (0.090 g), tris(dibenzylideneacetone) dipalladium (0) (0.028 g), Xantphos (0.052 g), Cesium carbonate (0.43 g) and 1,4-dioxane (3.0 mL) was heated at 100°C for 5 hours. The mixture was cooled to room temperature, diluted with ethyl acetate and filtered through a layer of Hi-Flo. The solvent was removed under reduced pressure to afford the title compound as a yellow gum, 0.46 g.
MS: ESI (+ve) (Method B): 493 (M+H)⁺, Retention time 3.8 min.

### Preparation 5b: [4-difluoromethoxy-2-ethyl-8-fluoro-3-(4-methoxycarbonylaminobenzyl)quinolin-5-yloxy]acetic acid

A solution of [4-difluoromethoxy-2-ethyl-8-fluoro-3-(4-methoxycarbonylaminobenzyl)quinolin-5-yloxy]acetic acid methyl ester (0.61 g) in methanol (6.4 mL) and water (0.64 mL) was treated with 5.0 M aqueous lithium hydroxide solution (1.6 mL) and the resulting solution was stirred at room temperature for 2 hours. The mixture was acidified by the addition of glacial acetic acid, concentrated under reduced pressure and partitioned between ethyl acetate and water. The organic phase was dried over magnesium sulfate and concentrated under reduced pressure. The residue was purified by preparative reverse-phase HPLC to afford title compound as a primrose solid, 0.032 g.
¹H NMR (CDCl₃): δ 1.10 (t, J = 7.4 Hz, 3H), 2.80 (q, J = 7.4 Hz, 2H), 3.60 (s, 3H), 4.20 (s, 2H), 4.85 (s, 2H), 6.90-6.95 (m, 3H), 7.20 (t, J = 75 Hz, 1H), 7.30 (d, J = 8.9 Hz, 1H), 7.45 (dd, J = 8.9, 10.1 Hz, 1H), 9.50 (s, 1H), 13.00 (br s, 1H).
MS: ESI (+ve) (Method A): 479 (M+H)⁺, Retention time 10.2 min.
MS: ESI (+ve) (Method B): 479 (M+H)⁺, Retention time 3.2 min.

### Example 6: [4-difluoromethoxy-2-ethyl-8-fluoro-3-(3-methanesulfonylbenzyl)quinolin-5-yloxy]acetic acid

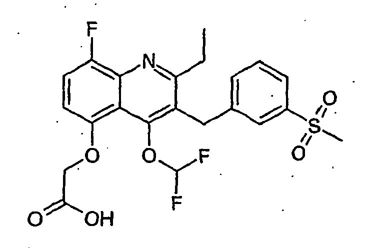

### Preparation 6a: 2-(3-methanesulfonylbenzyl)-3-oxopentanoic acid ethyl ester

The title compound was prepared by the method of Preparation 1 busing 3-oxopentanoic acid ethyl ester and 1-bromomethyl-3-methanesulfonylbenzene.
¹H NMR (CDCl₃): δ 1.05 (t, J = 7.3 Hz, 3H), 1.20 (t, J = 7.1 Hz, 3H), 2.40 (m, 1H), 2.65 (m, 1H), 3.05 (s, 3H), 3.25 (m, 2H), 3.80 (t, J = 7.4 Hz, 1H), 4.15 (m, 2H), 7.50 (m, 2H), 7.80 (m, 2H).

### Preparation 6b: [2-ethyl-8-fluoro-3-(3-methanesulfonylbenzyl)-4-oxo-1,4-dihydroquinolin-5-yloxy]acetic acid methyl ester

The title compound was prepared by the method of Preparation 1c using (3-amino-4-fluorophenoxy)acetic acid methyl ester and 2-(3-methanesulfonylbenzyl)-3-oxopentanoic acid ethyl ester.
MS: ESI (+ve) (Method B): 448 (M+H)⁺, Retention time 2.6 min.

### Preparation 6c: [4-difluoromethoxy-2-ethyl-8-fluoro-3-(3-methanesulfonylbenzyl)quinolin-5-yloxy]acetic acid methyl ester

The title compound was prepared by the method of Preparation 1d using [2-ethyl-8-fluoro-3-(3-methanesulfonylbenzyl)-4-oxo-1,4-dihydroquinolin-5-yloxy]acetic acid methyl ester and acetic acid chlorodifluoromethyl ester.
MS: ESI (+ve) (Method B): 498 (M+H)⁺, Retention time 3.6 min.

### Preparation 6d: [4-difluoromethoxy-2-ethyl-8-fluoro-3-(3-methanesulfonylbenzyl)quinolin-5-yloxy]acetic acid

A solution of [4-difluoromethoxy-2-ethyl-8-fluoro-3-(3-methanesulfonylbenzyl)quinolin-5-yloxy]acetic acid methyl ester (0.54 g) in methanol (10 mL) was treated with 1.0 M aqueous sodium hydroxide solution (6.0 mL) and the resulting solution was stirred at room temperature for 2 hours. The mixture was concentrated under reduced pressure, diluted with water and washed with dichloromethane. The aqueous phase was acidified by the addition of glacial acetic acid, extracted with dichloromethane and the combined extracts dried over magnesium sulfate. The solvent was removed under reduced pressure and the residue purified by preparative reverse-phase HPLC to afford title compound as a pale yellow solid, 0.27 g.
¹H NMR (CDCl₃): δ 1.25 (t, J = 7.4 Hz, 3H), 2.85 (q, J = 7.4 Hz, 2H), 3.00 (s, 3H), 4.45 (s, 2H), 4.80 (s, 2H), 6.80 (dd, J = 3.6, 8.7 Hz, 1H); 6.85 (t, J = 75 Hz, 1H), 7.30 (t, J = 9.3 Hz, 1H), 7.35 (d, J = 7.8 Hz, 1H), 7.45 (t, J = 7.8 Hz, 1H), 7.65 (s, 1H), 7.75 (d, J = 7.8 Hz, 1H).
MS: ESI (+ve) (Method A): 484 (M+H)⁺, Retention time 9.5 min.

### Example 7: (S)-2-[3-(4-chlorobenzyl)-4-difluoromethoxy-2-ethyl-8-fluoroquinolin-5-yloxy]propionic acid

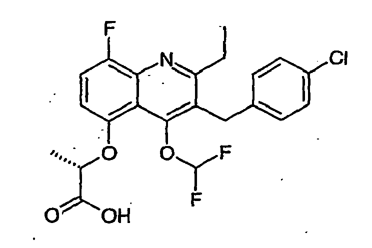

### Preparation 7a (S)-2-(3-amino-4-fluorophenoxy)propionic acid methyl ester

A solution of 3-amino-4-fluorophenol (4,1 g) in *N,N*-dimethylformamide (15 mL) was added dropwise to a stirred suspension of sodium hydride (60 % in oil, 1.3 g) in *N,N-*dimethylformamide (35 mL) at 0 °C. The mixture was stirred a room temperature for 30 minutes, cooled 0 °C and (*R*)-2-chloropropionic acid methyl ester (4.0 g) was added in one portion. The resulting mixture was stirred at room temperature overnight, treated with saturated aqueous ammonium chloride solution and extracted with ethyl acetate. The combined extracts were washed with saturated aqueous sodium chloride solution, dried over magnesium sulfate and concentrated under reduced pressure. Purification of the residue by column chromatography on silica gel, eluting with a mixture of dichloromethane and ethyl acetate (1:0 to 3:2 by volume) gave title compounds as golden oil, 2.5 g.
¹H NMR (CDCl₃): δ 1.55 (d, J = 6.7 Hz, 3H), 3.75 (s, 3H), 4.65 (q, J = 6.7 Hz, 1H), 6.15 (dt, J = 3.0, 8.8 Hz, 1H), 6.35 (dd, J = 3.0, 7.5 Hz, 1H), 6.85 (dd, J = 8.8, 10.6 Hz, 1H).

### Preparation 7b: 2-(4-chlorobenzyl)-3-oxopentanoic acid ethyl ester

A suspension of potassium *tert*-butoxide (7.8 g) in anhydrous tetrahydrofuran (140 mL) at 0 °C was treated with a mixture of *tert*-butanol (0.3 mL) and 3-oxopentanoic acid ethyl ester (10 g). After stirring at room temperature four 30 minutes a solution of 1-bromomethyl-4-chlorobenzene (14 g) in tetrahydrofuran (20 mL) was added and the resulting mixture stirred at room temperature for 6 days. The mixture was diluted with water, extracted with ethyl acetate and the combined extracts washed with water and saturated aqueous sodium chloride solution and then dried over magnesium sulfate. The solvent was removed under reduced pressure and the residue purified by column chromatography on silica gel, eluting with a mixture of pentane and ethyl acetate (1:0 to 10:1 by volume) to afford title compound as a yellow oil, 9.3 g.

### Preparation 7c: (S)-2-[3-(4-chlorobenzyl)-2-ethyl-8-fluoro-4-oxo-1,4-dihydroquinolin-5-yloxy]propionic acid methyl ester

The title compound was prepared by the method of Preparation 1c using (*S*)-2-(3-amino-4-fluorophenoxy)propionic acid methyl ester and 2-(4-chlorobenzyl)-3-oxopentanoic acid ethyl ester.

### Preparation 7d: (S)-2-[3-(4-chlorobenzyl)-4-difluoromethoxy-2-ethyl-8-fluoroquinolin-5-yloxy]propionic acid methyl ester

The title compound was prepared by the method of Preparation 1d using (*S*)-2-[3-(4-chlorobenzyl)-2-ethyl-8-fluoro-4-oxo-1,4-dihydroquinolin-5-yloxy]propionic acid methyl ester and acetic acid chlorodifluoromethyl ester.
¹H NMR (CDCl₃): δ 1.25 (t, J = 7.3 Hz, 3H), 1.75 (d, J = 6.7 Hz, 3H), 2.85 .(m, 2H), 3.75 (s, 3H), 4.20 (d, J = 16 Hz, 1H), 4.50 (d, J =16 Hz, 1H), 4.95 (q, J = 6.7 Hz, 1H), 6.60 (dd, J = 3.5, 8.6 Hz, 1H), 6.80 (dd, J = 69, 82 Hz, 1H), 7.00 (d, J = 8.4 Hz, 2H), 7.10-7.30 (m, 3H).

### Preparation 7e: (S)-2-[3-(4-chlorobenzyl)-4-difluoromethoxy-2-ethyl-8-fluoroquinolin-5-yloxy]propionic acid

A solution of (*S*)-2-[3-(4-chlorobenzyl)-4-difluoromethoxy-2-ethyl-8-fluoroquinolin-5-yloxy]propionic acid methyl ester (1.5 g) in industrial methylated spirits (10 mL) was treated with a solution of sodium hydroxide (0.32 g) in water ( 2.0 mL) and the resulting solution was stirred at room temperature for 1 hour. The mixture was partitioned between ethyl acetate and 2.0 M aqueous hydrochloric acid. The organic phase was washed with saturated aqueous sodium chloride solution, dried over magnesium sulfate and concentrated under reduced pressure. The residue was purified by column chromatography on silica gel, eluting with a mixture of toluene and glacial acetic acid (1:0 to 9:1 by volume) to give title compound as a yellow solid, 0.89 g.
¹H NMR (DMSO-d6): δ 1.20 (t, J = 7.4 Hz, 3H), 1.55 (d, J = 6.8 Hz, 3H), 2.75 (m, 2H), 4.30 (m, 2H), 5.10 (q, J = 6.8 Hz, 1H), 6.85 (dd, J = 3.7, 9.0 Hz, 1H), 7.05 (d, J = 8.6 Hz, 2H), 7.25 (dd, J = 72, 79 Hz, 1H), 7.30 (d, J = 8.6 Hz, 2H), 7.50 (dd, J = 9.0, 10.1 Hz, 1H), 13.30 (br s, 1H).
MS: ESI (+ve) (Method A): 453 (M+H)⁺, Retention time 12.9 min.

### Example 8: {4-difluoromethoxy-2-ethyl-8-fluoro-3-[4-(1-hydroxy-1-methylethyl)benzyl]quinolin-5-yloxy}acetic acid

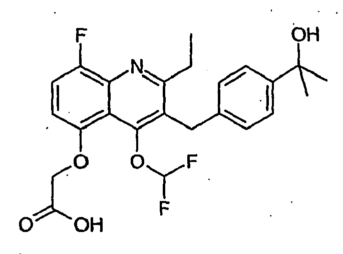

### Preparation 8a: 2-(4-acetylbenzyl)-3-oxopentanoic acid ethyl ester

The title compound was prepared by the method of Preparation 1 b using 3-oxobutyric acid ethyl ester and 1-(4-bromomethylphenyl)ethanone.
¹H NMR (CDCl₃): δ 1.00 (t, J = 7.2 Hz, 3H), 1.20 (t, J = 7.2 Hz, 3H), 2.35 (m, 1H), 2.55 (s, 3H), 2.60 (m, 1H), 3.20 (m, 2H), 3.80 (t, J = 7.5 Hz, 1H), 4.15 (m, 2H), 7.25 (d, J = 8.5 Hz, 2H), 7.85 (d, J = 8.5 Hz, 2H).

### Preparation 8b: [3-(4-acetylbenzyl)-2-ethyl-8-fluoro-4-oxo-1,4-dihydroquinolin-5-yloxy]acetic acid methyl ester

The title compound was prepared by the method of Preparation 1 c using (3-amino-4-fluorophenoxy)acetic acid methyl ester and 2-(4-acetylbenzyl)-3-oxopentanoic acid ethyl ester.
MS: ESI (+ve) (Method B): 412 (M+H)⁺, Retention time 2.9 min.

### Preparation 8c: [3-(4-acetylbenzyl)-4-difluoromethoxy-2-ethyl-8-fluoroquinolin-5-yloxy]acetic acid methyl ester

The title compound was prepared by the method of Preparation 1d using [3-(4-acetylbenzyl)-2-ethyl-8-fluoro-4-oxo-1,4-dihydroquinolin-5-yloxy]acetic acid methyl ester and acetic acid chlorodifluoromethyl ester.
MS: ESI (+ve) (Method B): 462 (M+H)⁺, Retention time 4.0 min.

### Preparation 8d: [3-(4-acetylbenzyl)-4-difluoromethoxy-2-ethyl-8-fluoroquinolin-5-yloxy]acetic acid

A solution of [3-(4-acetylbenzyl)-4-difluoromethoxy-2-ethyl-8-fluoroquinolin-5-yloxy]acetic acid methyl ester (0.13 g) in methanol (5.0 mL) was treated with 1.0 M aqueous lithium hydroxide solution (0.54 mL) and the resulting solution was stirred at room temperature for 30 minutes. The mixture was concentrated under reduced pressure, diluted with water and acidified by the addition of 0.1 M aqueous hydrochloric acid. The mixture was extracted with ethyl acetate and the combined extracts washed with saturated aqueous sodium chloride solution and dried over magnesium sulfate. The solvent was removed under reduced pressure and the residue purified by column chromatography on C-18 column to afford title compound, 0.20 g.
¹H NMR (CD₃OD) δ 1.15 (t, J = 7.5 Hz, 3H), 2.50 (s, 3H), 2.85 (q, J = 7.5 Hz, 2H), 4.45 (s, 2H), 4.85 (s, 2H), 6.90 (dd, J = 3.7, 8.8 Hz, 1H), 7:15 (t, J = 75 Hz, 1H), 7.20 (d, J = 8.3 Hz, 2H), 7.35 (dd, J = 8.8, 10.0 Hz, 1H), 7.90 (d, J = 8.3 Hz, 2H).
MS: ESI (+ve) (Method A): 448 (M+H)⁺, Retention time 10.4 min.
MS: ESI (+ve) (Method B): 448 (M+H)⁺, Retention time 3.7 min.

### Preparation 8e: {4-difluoromethoxy-2-ethyl-8-fluoro-3-[4-(1-hydroxy-1-methylethyl)benzyl]quinolin-5-yloxy}acetic acid

A solution of [3-(4-acetylbenzyl)-4-difluoromethoxy-2-ethyl-8-fluoroquinolin-5-yloxy]acetic acid (0.060 g) in tetrahydrofuran (5.0 mL) was treated with methylmagnesium bromide (3.0 M in diethyl ether, 0.58 mL) and the resulting mixture was heated at reflux for 29 hours. The mixture was cooled to room temperature, diluted with saturated aqueous ammonium chloride solution and extracted with ethyl acetate. The combined extracts were washed with saturated aqueous sodium chloride solution, dried over magnesium sulfate and concentrated under reduced pressure. The residue was purified by column chromatography on C-18 column to afford title compound, 0.011 g.
¹H NMR (DMSO-d6): δ 1.15 (t, J = 7.4 Hz, 3H), 1.35 (s, 6H), 2.80 (q, J = 7.4 Hz, 2H), 4.25 (s, 2H), 4.45 (s, 2H), 4.85 (s, 1H), 6.80 (dd, J = 3.7, 8.8 Hz, 1H), 6.95 (d, J = 8.3 Hz, 2H), 7.30 (d, J = 8.3 Hz, 2H), 7.40 (dd, J = 8.8, 10.1 Hz, 1H), 8.00 (t, J = 75 Hz, 1H).
MS: ESI (+ve) (Method A): 464 (M+H)⁺, Retention time 10.0 min.
MS: ESI (+ve) (Method B): 464 (M+H)⁺, Retention time 3.7 min.

### Example 9: [8-chloro-3-(4-cyanobenzyl)-4-difluoromethoxy-2-ethylquinolin-5-yloxy]acetic acid

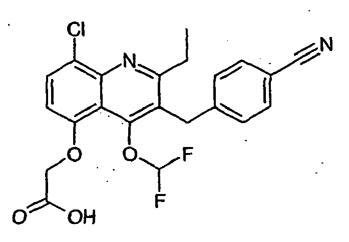

### Preparation 9a: (4-chloro-3-nitrophenoxy)acetic acid methyl ester

A mixture of 4-chloro'3-nitrophenof (25 g), *N,N*-dimethylformtmide (200 mL), potassium carbonate (60 g) and bromoacetic acid methyl ester (15.5 mL) was stirred at room temperature for 2.5 hours. The mixture was partitioned between ethyl acetate and water and the aqueous phase extracted with ethyl acetate. The combined extracts were dried over sodium sulfate and the solvent removed under reduced pressure. The residue was washed with diethyl ether to afford title compound as a white solid, 30 g.
¹H NMR (CDCl₃): δ 3.85 (s, 3H), 4.70 (s, 2H), 7.10 (dd, J = 3.0, 8.9 Hz, 1H), 7.40 (dd, J = 3.0 Hz, 1H), 7.45 (d, J = 8.9 Hz, 1H).

### Preparation 9b: (3-amino-4-chlorophenoxy)acetic acid methyl ester

A solution of (4-chloro-3-nitrophenoxy)acetic acid methyl ester (30 g) in methanol (100 mL) was added to a mixture of iron (26 g), ammonium chloride (33 g) and water (400 mL) at room temperature. The resulting mixture was heated in an ultrasonic bath at 60 °C for 4 hours. The mixture was basified by the addition of sodium hydroxide and then extracted with ethyl acetate. The combined extracts were washed with 1.0 M aqueous hydrochloric acid and the pH of the combined aqueous phases were adjusted to 7-8 by the addition of sodium hydroxide. The resulting precipitate was collected by filtration and dried to afford title compound, 14 g.
¹H NMR (DMSO-d6): δ 3.70 (s, 3H), 4.60 (s, 2H), 5.35 (br s, 2H), 6.10 (dd, J = 3.0, 8.8 Hz, 1H), 6.35 (d, J = 3.0 Hz, 1H), 7.05 (d, J = 8.8 Hz, 1H).

### Preparation 9c: [8-chloro-3-(4-cyanobenzyl)-2-ethyl-4-oxo-1,4-dihydroquinolin'-5-yloxy]acetic acid methyl ester

The title compound was prepared by the method of Preparation 1 c using (3-amino-4-chlorophenoxy)acetic acid methyl ester and 2-(4-cyanobenzyl)-3-oxopentanoic acid ethyl ester
MS: ESI (+ve) (Method B): 411 (M+H)⁺, Retention time 3.3 min.

### Preparation 9d: [8-chloro-3-(4-cyanobenzyl)-4-difluoromethoxy-2-ethylquinolin-5-yloxy]acetic acid methyl ester

To a mixture of [8-chloro-3-(4-cyanobenzyl)-2-ethyl-4-oxo-1,4-dihydroquinolin-5-yloxy]acetic acid methyl ester (0.19 g), *N,N* dimethylformamide (5.0 mL) and potassium carbonate (0.19 g) at -80 °C was added chlorodifluoromethane (0.4 mL). The flask was sealed and the resulting mixture warmed to room temperature and then stirred at 40 °C for 6 hours. The excess chlorodifluoromethane was allowed to evaporate and the residue partitioned between ethyl acetate and saturated aqueous sodium chloride solution. The organic phase was dried over magnesium sulfate and the solvent removed under reduced pressure. Purification of the residue by column chromatography on silica gel, eluting with a mixture of cyclohexane and ethyl acetate (1:0 to 1:1 by volume) gave title compound, 0.13 g.
MS: ESI (+ve) (Method B): 461 (M+H)⁺, Retention time 4.3 min.

### Preparation 9e: [8-chloro-3-(4-cyanobenzyl)-4-difluoromethoxy-2-ethylquinolin-5-yloxy]acetic acid

A solution of [8-chloro-3-(4-cyanobenzyl)-4-difluoromethoxy-2-ethylquinolin-5-yloxy]acetic acid methyl ester (0.13 g) in tetrahydrofuran (10 mL) was treated with 1.0 M aqueous lithium hydroxide solution (0.56 mL) and the resulting mixture was stirred at room temperature for 3 hours. The mixture was concentrated under reduced pressure, diluted-with water and the pH adjusted to 4-5 by the addition of 0.1 M aqueous hydrochloric acid. The mixture was extracted with ethyl acetate and the combined extracts dried over sodium sulfate and then concentrated under reduced pressure. The residue was purified by preparative reverse-phase HPLC to afford title compound, 0.087 g.
¹H NMR (CDCl₃): δ 1.35 (t, J = 7.4 Hz, 3H), 2.85 (q, J = 7.4 Hz, 2H), 4.35 (s, 2H), 4.90 (s, 2H), 6.75 (d, J = 8.5 Hz, 1H), 6.85 (t, J = 75 Hz, 1H), 7.20 (d, J = 8.2 Hz, 2H), 7.55 (d, J = 8.2 Hz, 2H), 7.75 (d, J =8.5 Hz, 1H).
MS: ESI (+ve) (Method A): 447 (M+H)⁺, Retention time 12.0 min.

### Example 10: [8-chloro-3-(2-chloro-4-cyanobenzyl)-4-difluoromethoxy-2-ethylquinolin-5-yloxy]acetic acid

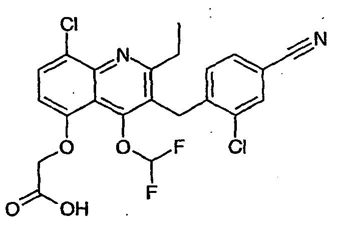

### Preparation 10a: 2-(2-chloro-4-cyanobenzyl)-3-oxopentanoic acid methyl ester

The title compound was prepared by the method of Preparation 1b using 3-oxopentanoic acid methyl ester and 4-bromomethyl-3-chlorobenzonitrile.
MS: ESI (+ve) (Method B): 280 (M+H)⁺, Retention time 3.6 min.

### Preparation 10b: [8-chloro-3-(2-chloro-4-cyanobenzyl)-2-ethyl-4-hydroxyquinolin-5-yloxy]acetic acid methyl ester

The title compound was prepared by the method of Preparation 1 c using (3-amino-4-chlorophenoxy)acetic acid methyl ester and 2-(2-chloro-4-cyanobenzyl)-3-oxopentanoic acid methyl ester.
MS: ESI (+ve) (Method B): 445 (M+H)⁺, Retention time 3.6 min.

### Preparation 10c: [8-chloro-3-(2-chloro-4-cyanobenzyl)-4-difluoromethoxy-2-ethylquinolin-5-yloxy]acetic acid methyl ester

The title compound was prepared by the method of Preparation 9d using [8-chloro-3-(2-chloro-4-cyanobenzyl)-2-ethyl-4-hydroxyquinolin-5-yloxy]acetic acid methyl ester and chlorodifluoromethane.
¹H NMR (CDCl₃): δ 1.35 (t, J = 7.3 Hz, 3H), 2.80 (q, J = 7.3 Hz, 2H), 3.85 (s, 3H), . 4.50 (s, 2H), 4.85 (s, 2H), 6.70 (d, J = 8.0 Hz, 1H), 6.75 (d, J = 8.5 Hz, 1H), 6.95 (t, J = 75 Hz, 1H), 7.35 (dd, J = 1.6, 8.0 Hz, 1H), 7.70 (d, J = 1.6 Hz, 1H), 7.70 (d, J = 8.5 Hz, 1H).

### Preparation 10d: [8-chloro-3-(2-chloro-4-cyanobenzyl)-4-difluoromethoxy-2-ethylquinolin-5-yloxy]acetic acid

A mixture of [8-chloro-3-(2-chloro-4-cyanobenzyl)-4-difluoromethoxy-2-ethylquinolin-5-yloxy]acetic acid methyl ester (0.22 g), tetrahydrofuran (8.0 mL), methanol (1.0 mL) and water (1.0 mL) was treated with 1.0 M aqueous lithium hydroxide solution (0.90 mL) and the resulting mixture was stirred at room temperature for 2 hours. The mixture was concentrated under reduced pressure, diluted with water and the pH adjusted to 4-5 by the addition of 0.1 M aqueous hydrochloric acid. The mixture was extracted with ethyl acetate and the combined extracts dried over magnesium sulfate and then concentrated under reduced pressure. The residue was purified by preparative reverse-phase HPLC to afford title compound, 0.11 g.
¹H NMR (CDCl₃): δ 1.35 (t, J = .7.2 Hz, 3H), 2.80 (q, J = 7.2 Hz, 2H), 4.45 (s, 2H), 4.85 (s, 2H), 6.70 (d, J = 8.7 Hz, 1H), 6.75 (d, J = 8.7 Hz, 1H), 6.85 (t, J = 75 Hz, 1H), 7.35 (dd, J = 1.6, 8.0 Hz, 1H), 7.70-7.75 (m, 2H).
MS: ESI (+ve) (Method A): 481 (M+H)⁺, Retention time 13.0 min.

### Example 11: [8-chloro-3-(4-cyanobenzyl)-2-difluoromethoxy-4-methylquinolin-5-yloxy]acetic acid

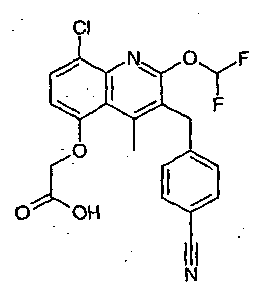

### Preparation 11a: 2-(4-cyanobenzyl)-3-oxothiobutyric acid S-tert-butyl ester

A solution of 3-oxothiobutyric acid *S*-terf-butyl ester (5.0 g) in 1,2-dimethoxyethane (20 mL) was added to a stirred suspension of sodium hydride (60 % in oil, 1.2 g) in 1,2-dimethoxyethane (50 mL) at -20 °C. The mixture was warmed to 0 °C for 15 minutes, cooled to -20 °C and then a solution of 4-bromomethylbenzonitrile (8.4 g) in 1,2-dimethoxyethane (60 mL) was added dropwise. The resulting mixture was warmed to room temperature over 45 minutes and then stirred at this temperature for 18 hours. The mixture was diluted with saturated aqueous ammonium chloride solution and the phases separated. The aqueous phase was extracted with ethyl acetate and the combined organic phases were dried over magnesium sulfate and concentrated under reduced pressure. Purification of the residue by column chromatography on silica gel, eluting with a mixture of cyclohexane and ethyl acetate (1:0 to 3:2 by volume) gave title compound, 7.1 g.
¹H NMR (CDCl₃): δ 1.40 (s, 9H), 2.25 (s, 3H), 3.15 (d, J = 8.1 Hz, 1H), 3.20 (d, J = 6.8 Hz, 1H), 3.80 (dd, J = 6.8, 8.1 Hz, 1H), 7.25 (d, J = 8.5 Hz, 2H), 7.55 (d, J = 8.5 Hz, 2H).

### Preparation 11b: N-(2-chloro-5-hydroxyphenyl)-2-(4-cyanobenzyl)-3-oxobutyramide

Silver trifluoroacetate (1.1 g) was added to a stirred solution of 3-amino-4-chlorophenol (0.56 g) and 2-(4-cyanobenzyl)-3-oxothiobutyric acid *S*-tert-butyl ester (0.95 g) in 1,2-dimethoxyethane (10 mL) at room temperature and the resulting mixture was stirred at room temperature overnight. The mixture was filtered through Hy-Flo, washing with 1,2-dimethoxyethane and then concentrated under reduced pressure. The residue was triturated with dichloromethane to give title compound as a brown solid, 0.52 g.
MS: ESI (+ve) (Method B): 343 (M+H)⁺, Retention time 2.9 min.

### Preparation 11 c: 4-(8-chloro-2,5-dihydroxy-4-methylquinolin-3-ylmethyl)benzonitrile

A mixture of *N*-(2-chloro-5-hydroxyphenyl)-2-(4-cyanobenzyl)-3-oxobutyramide (0.23 g), toluene (20 mL) and toluene-4-sulfonic acid monohydrate (0.26 g) was heated at 120 °C for 2 hours. The mixture cooled to room temperature, diluted with saturated aqueous sodium acetate solution and extracted with ethyl acetate. The combined extracts were dried over magnesium sulfate and the solvent removed under reduced pressure. The residue triturated with methanol to give title compound as a white solid, 0.16 g.
¹H NMR (DMSO-d6): δ 2.60 (s, 3H), 4.15 (s, 2H), 6.60 (d, J = 8.7 Hz, 1H), 7.40 (m, 3H), 7.75 (d, J = 8.1 Hz, 2H), 10.4 (br s, 1H).

### Preparation 11d: [8-chloro-3-(4-cyanobenzyl)-2-hydroxy-4-methylquinolin-5-yloxy]acetic acid methyl ester

The title compound was prepared by the method of Preparation 3c using 4-(8-chloro-2,5-dihydroxy-4-methylquinolin-3-ylmethyl)benzonitrile and bromoacetic acid methyl ester.
MS: ESI (+ve) (Method B): 397 (M+H)⁺, Retention time 3.5 min.

### Preparation 11e: [8-chloro-3-(4-cyanobenzyl)-2-difluoromethoxy-4-methylquinolin-5-yloxy]acetic acid methyl ester

A mixture of [8-chloro-3-(4-cyanobenzyl)-2-hydroxy-4-methylquinolin-5-yloxylacetic acid methyl ester (0.079 g), *N,N*-dimethylformamide (2.0 mL) and potassium carbonate (0.083 g) was stirred at 40 °C for 2.5 hours under an atmosphere of chlorodifluoromethane. The mixture was diluted with water, extracted with ethyl acetate and the combined extracts dried over magnesium sulfate. The solvent was removed under reduced pressure and the residue triturated with methanol to afford title compound, 0.075 g.
MS: ESI (+ve) (Method B): 447 (M+H)⁺, Retention time 4:4 min.

### Preparation 11f: [8-chloro-3-(4-cyanobenzyl)-2-difluoromethoxy-4-methylquinolin-5-yloxy]acetic acid

A solution of [8-chloro-3-(4-cyanobenzyl)-2-difluoromethoxy-4-methylquinolin-5-yloxy]acetic acid methyl ester (0.042 g) in tetrahydrofuran (3.0 mL) and water (0.5 mL) was treated with 1.0 M aqueous lithium hydroxide solution (0.34 mL) and the resulting mixture was stirred at room temperature for 2 hours. The mixture was concentrated under reduced pressure, diluted with water and the pH adjusted to 4-5 by the addition of 0.1 M aqueous hydrochloric acid. The mixture was extracted with ethyl acetate and the combined extracts dried over magnesium sulfate and then concentrated under reduced pressure. The residue was purified by preparative reverse-phase HPLC to afford title compound, 0.025 g.
¹H NMR (DMSO-d6): δ 2.80 (s, 3H), 4.30 (s; 2H), 4.80 (s, 2H), 6.95 (d, J = 8.5 Hz, 1H), 7.30 (d, J = 8.3 Hz, 2H), 7.70 (d, J = 8.3 Hz, 2H), 7.75 (d, J = 8.5 Hz, 1H), 7.80 (t, J = 72 Hz, 1H).
MS: ESI (+ve) (Method A): 433 (M+H)⁺, Retention time 12.0 min. :

### Example 12: [3-(4-cyanobenzyl)-2-difluoromethoxy-8-fluoro-4-methylquinolin-5-yloxy]acetic acid

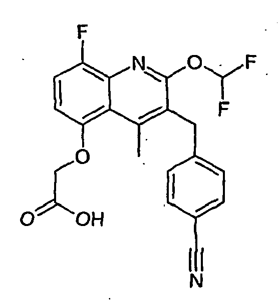

### Preparation 12a: 2-(4-cyanobenzyl)-N-(2-fluoro-5-hydroxyphenyl)-3-oxobutyramide

The title compound was prepared by the method of Preparation 11 busing 3-amino-4-chlorophenol and 2-(4-cyanobenzyl)-3-oxothiobutyric acid S-tert-butyl ester.
MS: ESI (+ve) (Method B): 327 (M+H)⁺, Retention time 2.8 min.

### Preparation 12b: 4-(8-fluoro-2,5-dihydroxy-4-methylquinolin-3-ylmethyl)benzonitrile

The title compound was prepared by the method of Preparation 11c using 2-(4-cyanobenzyl)-*N*-(2-fluoro-5-hydroxyphenyl)-3-oxobutyramide
¹H NMR (DMSO-d6): δ 2.60 (s, 3H), 4.10 (s, 2H), 6.50 (dd, J = 4.4, 8.7 Hz, 1H), 7.15 (m, 1H), 7.40 (d, J = 8.1 Hz, 2H), 7.75 (d, J = 8.1 Hz, 2H), 10.20 (br s, 1H), 11.50 (br s, 1H).

### Preparation 12c: [3-(4-cyanobenzyl)-8-fluoro-2-hydroxy-4-methylquino(in-5-yloxy]acetic acid methyl ester

The title compound was prepared by the method of Preparation 3c using: 4-(8-fluoro-2,5-dihydroxy-4-methylquinolin-3-ylmethyl)benzonitrile and bromoacetic acid methyl ester.
MS: ESI (+ve) (Method B): 381 (M+H)⁺, Retention time 3.2 min.

### Preparation 12d: [3-(4-cyanobenzyl)-2-difluoromethoxy-8-fluoro-4-methylquinolin-5-yloxy]acetic acid methyl ester

The title compound was prepared by the method of Preparation 11e using: [3-(4-cyanobenzyl)-8-fluoro-2-hydroxy-4-methylquinolin-5-yloxy]acetic acid methyl ester and chlorodifluoromethane.
MS: ESI (+ve) (Method B): 431 (M+H)⁺, Retention time 4.2 min.

### Preparation 12e: [3-(4-cyanobenzyl)-2-difluoromethoxy-8-fluoro-4-methylquinolin-5-yloxy]acetic acid :

A solution of [3-(4-cyanobenzyl)-2-difluoromethoxy-8-fluoro-4-methylquinolin-5-yloxy]acetic acid methyl ester (0.089 g) in tetrahydrofuran (3.0 mL) and water (1.0 mL) was treated with 1.0 M aqueous lithium hydroxide solution (0.42 mL) and the resulting mixture was stirred at room temperature for 1.5 hours. The mixture was concentrated under reduced pressure, diluted with water and the pH adjusted to 4-5 by the addition of 0.1 M aqueous hydrochloric acid, The mixture was extracted with ethyl acetate and the combined extracts dried over magnesium sulfate and then concentrated under reduced pressure. The residue was purified by preparative reverse-phase HPLC to afford title compound, 0.015 g.
NMR (DMSO-d6): δ 2.85 (s, 3H), 4.30 (s, 2H), 4.85 (s, 2H), 6.95 (dd, J = 4.0, 9.0 Hz, 1H), 7.35 (d, J = 8.4 Hz, 2H), 7.50 (dd, J = 9.0, 9.7 Hz, 1H), 7.75 (d, J = 8.4 Hz, 2H), 7.85 (t, J = 72 Hz, 1H).
MS: ESI (+ve) (Method A): 417 (M+H)⁺, Retention time 11.3 min.

### Biological Methods

Compounds of the invention were tested using the following biological test methods to determine their ability to displace PGD₂ from the CRTH2 receptor and for their ability to antagonise the functional effects of PGD₂ at the CRTH2 receptor.

### Radioligand Binding Assay

The receptor binding assay is performed in a final volume of 200 µL binding buffer [1,0 mM BES (pH 7.4), 1 mM EDTA, 10 mM manganese chloride, 0.01 % BSA] and 1 nM [³H]-PGD₂ (Amersham Biosciences UK Ltd). Ligands are added in assay buffer containing a constant amount of DMSO (1 % by volume). Total binding is determined using 1 % by volume of DMSO in assay buffer and non-specific binding is determined using 10 µM of unlabeled PGD₂ (Sigma). Human embryonic kidney (HEK) cell membranes (3.5 µg) expressing the CRTH2 receptor are incubated with 1.5 mg wheatgerm agglutinin SPA beads and 1 nM [³H]-PGD₂ (Amersham Biosciences UK Ltd) and the mixture incubated for 3 hours at room temperature. Bound [³H]-PGD₂ is detected using a Microbeta TRILUX liquid scintillation counter (Perkin Elmer). Compound IC₅₀ value is determined using a 6-point dose response curve in duplicate with a semi-log compound dilution series. IC₅₀ calculations are performed using Excel and XLfit (Microsoft), and this value is used to determine a Ki value for the test compound using the Cheng-Prusoff equation.

### Functional Assay

### GTPγS Assay

The GTPyS Assay is performed in a final volume of 200 mL assay buffer (20mM HEPES pH 7.4,10mM MgCl₂, 100mM NaCl, 10µg/mL saponin). DMSO concentrations are kept constant at 1% by volume. Human embryonic kidney (HEK) cell membranes (3.5 µg) expressing the CRTH2 receptor are incubated with the compounds for 15 min at 30° C prior to addition of PGD₂ (30nM final concentration) and GTP (10µM final concentration). The assay solutions are then incubated for 30 minutes at 30° C, followed by addition of [³⁵S]-GTPγS (0.1nM final concentration). The assay plate is than shaken and incubated for 5 minutes at 30°C. Finally, SPA beads (Amersham Biosciences, UK) are added to a final concentration of 1.5mg/wett and the plate shaken and incubated for 30 minute at 30° C. The sealed plate is centrifuged at 1000g for 10mints at 30oC and the bound [³⁵S]-GTPγS is detected on Microbeta scintillation counter (Perkin Elmer). Compound IC₅₀ value is determined using a 6-point dose response curve in duplicate with a semi-log compound dilution series. IC₅₀ calculations are performed using Excel and XLfit (Microsoft), and this value is used to determine a Ki value for the test compound using the Cheng-Prusoff equation.

### Biological Results:

The compounds of the Examples above were tested in the CRTH2 radioligand binding and GTPγS functional assays described above; the compounds all have IC₅₀ values of less than 1 µM in both assays. For example, the compound of Example 1 had an IC₅₀ value of 6.1 nM in the CRTH2 radioligand binding assay, and the compound of Example 2 had an IC₅₀ value of 7.5 nM in that assay.

## Claims

1. A compound selected from the group consisting of:
[3-(4-cyanobenzyl)-4-difluoromethoxy-2-ethyl-8-fluoroquinolin-5-yioxy]acetic acid,
[3-(4-bromobenzyl)-4-difluoromethoxy-2-ethyl-8-fluoroquinolin-5-yloxy]acetic acid,
[4-ethyl-8-fluoro-3-(4-methanesulfonylbenzyl)-2-methylquinolin-5-yloxy]acetic acid,
[2-ethyl-8-fluoro-3-(4-methanesulfonylbenzyl)-4-methylquinolin-5-yloxy]acetic acid,
[4-difluoromethoxy-2-ethyl-8-fluoro-3-(4-methoxycarbonylaminobenzyl)quinolin-5-yloxy]acetic acid,
[4-difluoromethoxy-2-ethyl-8-fluoro-3-(3-methanesulfonylbenzyl)quinolin-5-yloxy]acetic acid,
(*S*)-2-[3-(4-chlorobenzyl)-4-difluoromethoxy-2-ethyl-8-fluoroquinolin-5-yloxy]propionic acid,
{4-difluoromethoxy-2-ethyl-8-fluoro-3-[4-(1-hydroxy-1-methylethyl)benzyl]quinolin-5-yloxy)acetic acid,
[8-chloro-3-(4-cyanobenzy!)-4-difluoromethoxy-2-ethylquinolin-5-yloxy]acetic acid,
[8-chloro-3-(2-chloro-4-cyanobenzyl)-4-difluoromethoxy-2-ethylquinolin-5-yloxy]acetic acid,
[8-chloro-3-(4-cyanobenzyl)-2-difluoromethoxy-4-methylquinolin-5-yloxy]acetic acid,
[3-(4-cyanobenzyl)-2-difluoromethoxy-8-fluoro-4-methylquinolin-5-yloxy]acetic acid,
and salts, N-oxides, hydrates and solvates thereof.

2. A pharmaceutical composition comprising a compound as claimed in claim 1 and a pharmaceutically acceptable carrier.

3. A compound as claimed in claim 1, for use in therapy.

4. A compound for use as claimed in claim 3, wherein the therapy is the treatment of asthma, chronic obstructive pulmonary disease, rhinitis, allergic airway syndrome, or allergic rhinobronchitis.

5. A compound for use as claimed in claim 3, wherein the therapy is the treatment of psoriasis, atopic and non-atopic dermatitis Crohn's disease, ulcerative colitis or irritable bowel disease.

## Patentansprüche

1. Eine Verbindung, die aus der Gruppe ausgewählt ist, die umfasst:
[3-(4-Cyanobenzyl)-4-difluormethoxy-2-ethyl-8-fluorchinolin-5-yloxy]essigsäure,
[3-(4-Brombenzyl)-4-difluormethoxy-2-ethyl-8-fluorchinolin-5-yloxy]essigsäure,
[4-Ethyl-8-fluor-3-(4-methansulfonylbenzyl)-2-methylchinolin-6-yloxy]essigsäure,
[2-Ethyl-8-fluor-3-(4-methansulfonylbenzyl)-4-methylchinolin-5-yloxy]essigsäure,
[4-Difluormethoxy-2-ethyl-8-fluor-3-(4-methoxycarbonylaminobenzyl)chinolin-5-yloxy]essigsäure,
[4-Difluormethoxy-2-ethyl-8-fluor-3-(3-methansulfonylbenzyl)chinolin-5-yloxy]essigsäure,
(*S*)-2-[3-(4-Chlorbenzyl)-4-difluormethoxy-2-ethyl-8-fluorchinolin-5-yloxy]propionsäure,
{4-Difluormethoxy-2-ethyl-8-fluor-3-[4-(1-hydroxy-1-methylethyl)benzyl]chinolin-5-yloxy}essigsäure,
[8-Chlor-3-(4-cyanobenzyl)-4-difluormethoxy-2-ethylchinolin-5-yloxy]essigsäure,
[8-Chlor-3-(2-chlor-4-cyanobenzyl)-4-difluormethoxy-2-ethylchinolin-5-yloxy]essigsäure,
[8-Chlor-3-(4-cyanobenzyl)-2-difluormethoxy-4-methylchinolin-5-yloxy]essigsäure,
[3-(4-Cyanobenzyl)-2-difluormethoxy-8-fluor-4-methylchinolin-5-yloxy]essigsäure,
und Salze, N-Oxide, Hydrate und Solvate derselben.

2. Eine pharmazeutische Zusammensetzung, die eine Verbindung gemäß Anspruch 1 und eine pharmazeutisch akzeptable Trägersubstanz umfasst.

3. Eine Verbindung gemäß Anspruch 1 zur Verwendung in der Therapie.

4. Eine Verbindung zur Verwendung gemäß Anspruch 3, wobei die Therapie die Behandlung von Asthma, chronisch obstruktiver Lungenerkrankung, Rhinitis, allergischem Atemwegssyndrom oder allergischer Rhinobronchitis ist.

5. Eine Verbindung zur Verwendung gemäß Anspruch 3, wobei die Therapie die Behandlung von Psoriasis, atopischer und nicht atopischer Dermatitis, Morbus Crohn, Colitis ulcerosa oder Reizdarmsyndrom ist.

## Revendications

1. Composé sélectionné parmi le groupe constitué par :
l'acide [3-(4-cyanobenzyl)-4-difluorométhoxy-2-éthyl-8-fluoroquinoline-5-yloxy] acétique,
l'acide [3-(4-bromobenzyl)-4-difluorométhoxy-2-éthyl-8-fluoroquinoline-5-yloxy] acétique,
l'acide [4-éthyl-8-fluoro-3-(4-méthanesulfonylbenzyl)-2-méthylquinoline-5-yloxy] acétique,
l'acide [2-éthyl-8-fluoro-3-(4-méthanesulfonylbenzyl)-4-méthylquinoline-5-yloxy] acétique,
l'acide [4-difluorométhoxy-2-éthyl-8-fluoro-3-(4-méthoxycarbonylaminobenzyl)quinoline-5-yloxy] acétique,
l'acide [4-difluorométhoxy-2-éthyl-8-fluoro-3-(3-méthanesulfonylbenzyl)quinoline-5-yloxy] acétique,
l'acide (S)-2-[3-(4-chlorobenzyl)-4-difluorométhoxy-2-éthyl-8-fluoroquinoline-5-yloxy] propionique,
l'acide [4-difluorométhoxy-2-éthyl-8-fluoro-3-[4-(1-hydroxy-1-méthyléthyl)benzyl]quinoline-5-yloxy] acétique,
l'acide [8-chloro-3-(4-cyanobenzyl)-4-difluorométhoxy-2-éthylquinoline-5-yloxy] acétique,
l'acide [8-chloro-3-(2-chloro-4-cyanobenzyl)-4-difluorométhoxy-2-éthylquinoline-5-yloxy] acétique,
l'acide [8-chloro-3-(4-cyanobenzyl)-2-difluorométhoxy-4-méthylquinoline-5-yloxy] acétique,
l'acide [3-(4-cyanobenzyl)-2-difluorométhoxy-8-fluoro-4-méthylquinoline-5-yloxy] acétique,
et des sels, des N-oxydes, des hydrates et des solvates de celui-ci.

2. Composition pharmaceutique comprenant un composé selon la revendication 1 et un excipient pharmaceutiquement acceptable.

3. Composé selon la revendication 1, à utiliser en thérapie.

4. Composé à utiliser selon la revendication 3, dans lequel le traitement est le traitement de l'asthme, de la bronchopneumopathie chronique obstructive, de la rhinite, du syndrome respiratoire allergique ou de la rhinobronchite allergique.

5. Composé à utiliser selon la revendication 3, dans lequel le traitement est le traitement du psoriasis, de la dermatite atopique et non atopique, de la maladie de Crohn, de la colite ulcéreuse ou du syndrome du côlon irritable.
